# EUROPEAN PATENT SPECIFICATION

(11) **EP 2 327 315 B1**
(45) Date of publication and mention of the grant of the patent: **09.10.2013**
(21) Application number: 09177422.4
(22) Date of filing: 29.11.2009
(51) Int. Cl.: A23L 1/305, A23L 1/29

(54) **Dosing protocols for increasing protein synthesis in an active individual**
Dosierungsprotokolle zur Steigerung der Proteinsynthese in einer aktiven Person
Protocoles de dosage pour augmenter la synthèse des protéines chez un individu actif

(43) Date of publication of application: 01.06.2011
(73) Proprietor: Nestec S.A., 1800 Vevey (CH)
(72) Inventor: Zaltas, Eric, 1071 St.-Saphorin (CH); Moore, Daniel, Ryan, 1000 Lausanne (CH); Stellingwerff, Trent, 1052 Le Mont-sur-Lausanne (CH); Burke, Louise Mary, Melbourne, Victoria 3000, (CH); Hawley, John, Alan, Melbourne, Victoria 3000, (CH)
(74) Representative: Corticchiato, Olivier

(56) References cited:
- WO-A1-01/67895
- WO-A2-2006/042222
- US-A1- 2002 006 907
- US-A1- 2002 044 988
- US-A1- 2005 031 671
- US-A1- 2006 275 506
- US-B1- 6 248 375

## Description

### BACKGROUND

The present disclosure generally relates to methods for providing nutrition. More specifically, the present disclosure is directed to methods for providing nutritional products for increasing protein synthesis in an active individual.

Nutritional products for providing protein to a consumer are known. Typically, these nutritional products are not associated with any recommended intake requirements based on the types of consumers or activities of the consumers. For example, an athlete who engages in endurance activities will likely need to eat a greater amount (e.g. kcals of energy) of the nutritional product than a person going for a casual walk. As a result, a person who eats the nutritional products does not know how much of the nutritional product to consume in order to optimize the protein providing contents of the nutritional product. WO2006/042222 A2 discloses a carbonated protein beverage composition which provides a relatively high protein content.

### SUMMARY

The present disclosure provides various methods for providing nutritional products for protein intake before, during and after exercising to increase protein synthesis in an individual during a recovery period after the exercising. In a general embodiment, the present disclosure provides a method of providing nutrition to an athlete. The method comprises providing a protein-based product and providing personalized guidelines for consuming the protein-based product, which provide a recommended amount of the protein-based product to consume for an exercise regimen based on one or more characteristics of the athlete, and/or one or more training regimens of the athlete wherein the athlete consumes the protein-based product according to the personalized guidelines.

In an embodiment, the protein-based product comprises a protein blend comprising soy protein isolates, whey protein isolates and calcium caseinate, or any individual amino acid or whole protein. In an embodiment, the protein-based products consist essentially of compositions having the same protein profile but differing in dose. The protein-based products can be products having the same amino acid profile but differing in dose.

In an embodiment, the personalized guidelines recommend a bolus feeding dose in close timing to the exercise bout, e.g. before or after the exercise regimen. In another embodiment, the personalized guidelines recommend a pulse feeding dose before beginning, throughout, and after the exercise regimen.

In an embodiment, the characteristic of the athlete can be weight, height, age, gender or a combination thereof. The training regimen can be aerobics, weight-lifting, running, swimming, cycling, team-sports or a combination thereof.

In an embodiment, the personalized guidelines are further based on the duration of the training program of the athlete. The personalized guidelines can be determined based on a sport. The training program can be designed to achieve an objective such as weight-loss, muscle building, endurance training, muscle toning or a combination thereof. The personalized guidelines can provide the recommended amount of the protein-based product to consume before and during exercising based on at least one of the characteristic of the athlete and the training program of the athlete.

In an aspect the bite-size nutritional product has a sweet taste and is highly concentrated in protein. The protein-based product can include a coating. In an embodiment, the coating is chocolate. The coating can further include particulates.

In an embodiment, the protein-based product weighs from about 5 grams to 35. The protein-based product can have a protein content of about 2 grams to about 4 grams which is desirable for pulse dosing. The protein-based product can have a protein blend including soy protein isolates, whey protein isolates and calcium caseinate.

In an aspect the protein-based product includes an amount of protein ranging from about 20% to about 40% by weight of the protein-based product. The protein-based product can also include an amount of fat ranging from about 10% to about 40% by weight of the protein-based product. In a further aspect the protein-based product includes an amount of protein above 15% by weight together with less than 10% saturated fat by weight of the protein-based product.

In an alternative embodiment, the present disclosure provides a method of increasing the benefit of resistance exercise. The method comprises providing at least two different doses of a protein composition that has substantially the same amino acid profile and providing guidelines as to which dose to take pre-exercise, during exercise, and post exercise wherein the doses of protein composition are consumed according to the guidelines.

The present disclosure provides a method of selling a protein-based product. The method comprises providing one or more protein-based products in a single convenient package and providing personalized guidelines for consuming the protein-based products. The personalized guidelines provide the recommended amount of the protein-based product to consume before, during and after exercising. The method further comprises selling the protein-based product package and the guidelines to an athlete. The personalized guidelines can be based on at least one of a characteristic of the athlete and a training regimen of the athlete.

In one aspect, the protein-based products are not individually wrapped. In another embodiment, the protein-based products are individually wrapped.

In an alternative aspect, the present disclosure provides a method of improving protein synthesis of an athlete after exercising. The method comprises providing a protein-based product and providing personalized guidelines for consuming the protein-based product. The personalized guidelines provide the recommended amount of the protein-based product to consume before, during and after exercising based on at least one of a characteristic of the athlete and a training regimen of the athlete.

An advantage of the present disclosure is to provide improved methods of providing nutritional products before, during and after exercising.

Another advantage of the present disclosure is to provide new methods for consuming protein before, during and after exercising.

Still another advantage of the present disclosure is to provide an improved method for improving protein synthesis of an athlete after exercising.

Additional features and advantages are described herein, and will be apparent from, the following Detailed Description and the figures.

### BRIEF DESCRIPTION OF THE FIGURES

FIG. 1 summarizes the time course of the interventions and sample collections used in our protocol.

FIG. 2 show the amino acid profiles achieved by the nutritional interventions during various trials.

FIG. 3 shows fractional synthetic rates for mixed muscle protein synthesis for 5 hours following resistance exercise (* Placebo < Slow, Fast).

FIG. 4 is a schematic representation of the targeted proteins in the insulin-like growth factor (anabolic) signaling pathway.

FIG. 5 shows mTOR (A) and PRAS40 (B) phosphorylation at rest (1) prior to resistance training, and following 1 hour (2) and 5 hours post-exercise recovery for *placebo* ([P] white bars)*, fast delivery* ([F] blue bars) and *slow delivery* ([S] red bars).

FIG. 6 shows p70 S6 kinase ^{thr421/ser424} (A) and p70 S6 kinase ^{thr389} (B) phosphorylation at rest (1) prior to resistance training, and following 1 hour (2) and 5 hours post-exercise recovery for *placebo* ([P] white bars), *fast delivery* ([F] blue bars) and *slow delivery* ([S] red bars).

### DETAILED DESCRIPTION

The present disclosure relates generally to methods for providing nutrition to an athlete or an active individual. Nutritional products including protein(s) can be specifically designed and provided to an athlete before, during and after exercising. The nutritional products can have a sweet taste and be highly concentrated in protein. In addition, the nutritional products can meet both qualitative (e.g. a tri-source protein blend) and quantitative (e.g. fixed and regular amounts of protein per bite-size or a specific type of protein, e.g., whey protein isolate versus soy protein) protein requirements.

In a general embodiment, the present disclosure provides a method of providing nutrition to an athlete using customized or personal guidelines in conjunction with protein-based products. The method comprises providing a protein-based product and providing personalized guidelines for consuming the protein-based product. The personalized guidelines provide the recommended amount (e.g. total quantity) of the protein-based product to consume for an exercise regimen based on one or more characteristics of the athlete and/or one or more training regimens of the athlete.

As used herein, an athlete is any individual engaged in a physical activity (e.g., resistance exercise, sport). As used herein, the term "exercise regimen" means any physically exerting activity carried out for a specified duration by an athlete.

The personal guidelines can specifically recommend the amount of the protein-based product to consume at discrete time intervals before beginning the exercise regimen, during the exercise regimen and after the exercise regimen. The personal guidelines can also be based on the duration of the training regiment of the athlete.

It was surprisingly found that bolus and pulse feeding amounts of protein given to an athlete before and/or throughout exercising, respectively, were both effective in increasing protein synthesis after exercise. For example, the results of studies showed that the feeding of protein prior to exercise may have unexpected effects on the post-exercise delivery of amino acids that may not be theoretically ideal for optimal protein synthesis.

In an embodiment, the personal guidelines recommend a bolus feeding amount before and after the exercise regimen. As used herein, a bolus feeding amount is a single amount or dose of a protein-based product. The personal guidelines can recommend how much time prior to exercising the bolus feeding amount should be consumed. The bolus feeding amount can be a specific large quantity such the athlete does not need to consume any of the protein-based product during the exercising.

In another embodiment, the personal guidelines recommend a pulse feeding amount before beginning, throughout, and after the exercise regimen. As used herein, a pulse feeding amount is a repeated amount of a protein-based product. The personal guidelines can recommend the discrete time intervals before, during and after exercising that the protein-based products should be consumed by the athlete. The pulse feeding amount can be a specific small quantity such the athlete does not overeat the protein-based product throughout the exercise. The pulse feeding can result in a prolonged amino acid curve.

The protein-based products can be sized for personalized and optimal dosing of protein for providing protein to the athlete based on the size and structure of the nutritional products. For example, the athlete can consume a specified number of protein-based products to provide the right amount of protein to the athlete as set forth in the personalized guidelines. In this manner, the portions of the protein-based product consumed by the athlete can be easily determined and monitored. In an embodiment, the intake amount (e.g. amount consumed) is guided by the characteristic(s) of the athlete, and the personalized guidelines specify the dosage amounts of the protein-based products for the athlete to consume before, during and after exercising.

To prepare the guidelines and determine what guidelines should be followed, the one or more characteristics of the athlete can be, by way of example, weight, height, age, gender or a combination thereof. Any other physical characteristics of the athlete can also be used for determining the guidelines. The one or more training regimens can include, by way of example, aerobics, weight-lifting, running, swimming, cycling, team sports or any other suitable individual or group sports. The training regimen(s) can also be determined based on any type of sport (e.g. baseball, football, basketball, soccer, etc.).

The training regimen(s) can be determined based a personal objective of the athlete or consumer of the protein-based products. For example, the personal objective can be weight-loss, muscle building, optimal recovery and adaptation to training and/or a combination thereof.

In an embodiment, the personalized guidelines provide the recommended amount of the protein-based product for the athlete to consume before, during and after exercising based on at least one of the characteristic of the athlete and the training regimen of the athlete. The size of the protein-based product further provides ease of handling and dosing amount determination before, during and after the exercising.

In an embodiment, the protein-based product includes a coating. The coating can be provided for convenient and non-sticky handling of the protein-based product before, during and after consumption. The coating can include chocolate, for example, in the form of a chocolate shell to prevent shape loss. In an alternative embodiment, the coating can include particulates.

The coating can include two different chocolates for visual differentiation. The coating can include different flavors such peanut butter, cookie dough and vanilla yoghurt. The coating can be added to a protein/fat core using any suitable methods such as, for example, panning or enrobing.

The protein-based product can have any suitable shape for ease of handling. In an embodiment, each of the protein-based products weighs from about 0.1 grams to about 50 grams such as 2 grams, 4 grams, 6 grams, 8 grams, 10 grams, 12 grams, 14 grams, 16 grams, 18 grams, 20 grams, 22 grams, 24 grams, 26 grams, 28 grams, 30 grams, 32 grams, 34 grams, 36 grams, 38 grams, 40 grams, 42 grams, 44 grams, 46 grams, 48 grams and the like. In another embodiment, each of the protein-based products weighs from about 5 grams to about 25 grams. Preferably, each protein-based product can include a weight ranging from about 5 grams to about 15 grams. More preferably, each protein-based product can include a weight ranging from about 8 grams to 9 grams. A typical serving can include, for example, several of the protein-based products, which can provide a total of about 250 to about 320 kcal.

It is to be understood that individual values for the protein-based product weight can also be the definition of the limit of a range. For example, the disclosure of protein-based product weights of 22 grams and 28 grams is also to be regarded as the disclosure of the weight range from 22 grams to 28 grams. The same applies to any combination of specifically mentioned values through the present disclosure.

In another embodiment, the protein-based product can be administered intermittently, or as repeated pulses. For example, the protein-based product can be administered in an amount such as 6 x 5 grams or 10 x 3 grams throughout a specified period of time.

The distinct size of the protein-based products can provide ease of portability and convenience to the protein-based products. In addition, as previously discussed, the size can make it more convenient for the protein-based product to be eaten in discrete portion control amounts depending on the guidelines for the athlete. For example, the servings or the amount of the protein-based products that the athlete should consume before, during and after exercising can be easily adjusted by altering the number of protein-based products needed for a specific protein requirement according to the guidelines.

The protein-based product can contain a discrete amount of protein in one or more bite-size products to provide any suitable amount of protein to an athlete. The percentage of energy (e.g. in the form of calories) coming from protein can range up to 30%.

In an embodiment, the protein-based product includes an amount of protein ranging from about 20% to about 40% by weight of the protein-based product. In another embodiment, the protein-based product includes an amount of protein of about 30% by weight of the protein-based product. Moreover, the protein-based product can be made so that there is a consistent and countable quantity of protein per single protein-based product, for example, between about 2 grams to about 4 grams per protein-based product. Preferably, the protein-based product includes a protein content of about 2 grams to about 2.5 grams.

In an embodiment, the protein-based product can be in the form of a ready-to-drink ("RTD") beverage. In this regard, the RTD beverage can include from about 1% to about 20% by weight of protein such as 2%, 3%, 4%, 5%, 6%, 7%, 8%, 9% and the like. The RTD beverage could also be in a container having a large cap that can be used to accurately titrate out liquid protein doses.

The protein-based product can also contain a discrete amount of fat in one or more bite-size products to provide any suitable amount of energy to an athlete. For example, each of the products can provide a fat amount up to 9g/300 cal. Each of the products can also provide a saturated fat amount up to 4g/300 cal. In an embodiment, the percentage of energy (e.g. in the form of calories) coming from fat can be up to about 25%.

In an embodiment, the protein-based product includes an amount of fat ranging from about 10% to about 40% by weight of the protein-based product. Preferably, the protein-based product includes an amount of fat of about 30% by weight of the protein-based product.

The fat source in the nutritional product can further provide an improved mouth feel. Any fat source is suitable. For example, animal or plant fats may be used. To increase the nutritional value, n3-unsaturated and n6-unsaturated fatty acids may comprise the fat source. The fat source may also contain long chain fatty acids and/or medium chain fatty acids. For example, milk fat, canola oil, almond butter, peanut butter, corn oil and/or high-oleic acid sunflower oil may be used.

Specific ranges of the protein amounts in combination with the fat amounts of the protein-based product can also be advantageous in providing precise protein requirements to the athlete. In an embodiment, the protein-based product includes an amount of protein above 15% by weight together with an amount of saturated fat that is less than 10% by weight of the protein-based product.

Any suitable proteins or blends of proteins can be used. For example, the protein-based product can have a protein blend including soy protein isolates, whey protein isolates and calcium caseinate. An example of the protein blend is the Tri-source™ protein blend.

The presence of proteins in the nutritional product of the present disclosure has the advantage in that it is possible to provide an athlete with a more complete nutrition or protein synthesis before, during and after performance. For the protein source, any suitable dietary protein may be such as, for example, animal proteins (e.g. milk proteins, meat proteins and egg proteins); vegetable proteins (e.g. soy protein, wheat protein, rice protein, and pea protein); mixtures of free amino acids; or combinations thereof. Milk proteins, such as casein and whey milk proteins, and soy proteins are particularly preferred.

The proteins may be intact or hydrolysed or a mixture of intact and hydrolysed proteins. It may be desirable to supply partially hydrolysed proteins (degree of hydrolysis between 2 and 20%), for example, for athletes believed to be at risk of developing cows' milk allergy. Generally, at least partially hydrolysed proteins are easier and faster to metabolize by the body. This is in particular true for amino acids. In an embodiment, the protein-based product contains single/essential amino acids such as, for example, L-leucine, L-valine and/or L-isoleucine.

The protein-based products in certain embodiments can be made using any suitable process (e.g. cold-extrusion). For example, the protein-based products can be made using a three step manufacturing process involving: 1) bite forming, 2) product handling after forming and 3) product enrobing (e.g. with chocolate or sugar coating).

To produce bite-size individual pieces, the product forming step can include making a roll of a protein/fat mass and cutting it into small pieces and forming them into spheres. Once formed, the product can be handled before enrobing (e.g. coating). Cooling down of the product is recommended to avoid product cold-flow. For example, special handling can be used to avoid the products from sticking together. The products can also be enrobed very quickly to avoid water picking and shape loss.

An applied coating can be a way to prevent stickiness, hold the shape of the center mass and provide a hard, shiny and non-melting ball. Different coating methods are possible such as a chocolate panning (e.g. for hard core) and hard sugar panning (e.g. for softer core).

In another embodiment, the present disclosure provides a method of selling a protein-based product. The method comprises providing one or more protein-based products in a package and providing personalized guidelines for consuming the protein-based products. The personalized guidelines provide the recommended amount of the protein-based product to consume before, during and after exercising. The method further comprises selling the protein-based product package and the guidelines to an athlete. The personalized guidelines are based on one or more characteristics of the athlete and/or one or more training regimens of the athlete.

In an embodiment, the protein-based products are not individually wrapped. In another embodiment, the protein-based products are individually wrapped. There can be any suitable number of protein-based products in each package.

A nutritional kit can include one or more protein-based products and personalized guidelines on how many of the protein-based products an athlete should consume before, during and after exercising. A plurality of the protein-based products and the personalized guidelines can be kept together in a package.

The personalized guidelines of the nutritional kit can provide the recommended amount of the protein-based product to consume before, during and after exercise based on the body weight or other physical characteristics (e.g. weight, age, gender) of the athlete. In another embodiment, the personalized guidelines of the nutritional kit can provide the recommended amount of the protein-based product to the athlete before, during and after exercising based on the training regimen of the athlete.

In an alterative embodiment, the present disclosure provides a method of improving protein synthesis an athlete after exercising is complete. The method comprises providing a protein-based product and providing personalized guidelines for consuming the protein-based product. The personalized guidelines provide the recommended amount of the protein-based product to consume before, during and after exercising based on at least one of a characteristic of the athlete and a training regimen of the athlete.

### EXAMPLES

By way of example and not limitation, the following examples are illustrative of various embodiments of the present disclosure.

### EXAMPLE 1

Dietary protein sources have been described as "fast" or "slow" depending on the rate of digestion and absorption of amino acids into the blood. It is of interest to determine whether the pattern of delivery of amino acids from food sources, independent of the content of individual amino acids in the food, affects the protein synthesis response to resistance exercise. In sedentary populations, the alteration of amino acid availability achieved by differences in the rate of digestion of food proteins was found to alter postprandial protein retention. In young, healthy subjects, a "slow" protein (e.g., casein) meal achieved higher leucine balance than a "fast" protein (e.g., whey) meal matched for leucine content. This difference was not seen in elderly subjects. When meals were matched for total nitrogen content, elderly subjects showed greater nitrogen balance with the fast protein than the slow protein, compared with the young group.

It is hard to translate the results of this study into nutrition guidelines for athletes. It is difficult to determine the individual roles and interaction of the different amino acid content of protein-rich foods and the digestibility of food proteins. In addition, the acute effects of an exercise bout and the chronic effect of training need to be taken into account. In research involving the glycemic index ("GI") of carbohydrate foods, the model used to isolate the pattern of delivery of substrate to the muscle is to compare the responses to a high GI carbohydrate food consumed in a bolus (i.e., fast release of glucose) to consumption of the same food as a series of small snacks over the same time period (i.e., mimicking the low and sustained release of a low GI carbohydrate). This model achieves the differences in blood glucose and insulin responses seen with low and GI carbohydrate foods while providing an identical nutritional profile. This same idea was used to compare the response to a fast protein or slow protein consumed in relation to resistance exercise.

### Objective

The aim of this study was to investigate the role of amino acid availability as a regulator of net muscle protein synthesis before and during recovery from exercise in trained subjects, by manipulating the changes in the blood amino acid levels before and before and during exercise. Our model achieved a different pattern of amino acid delivery mimicking a "fast" (e.g., bolus) and "slow" (e.g., pulse) protein source while providing an identical amino acid concentration. Because of previous research that showed that benefits of amino acid feedings were greater when consumed prior to, rather than after the session, the protein sources were consumed pre-exercise. It is believed that post-exercise feeding may be as effective as.

### Hypotheses

1. In comparison to a control (fasted) trial, trials involving the intake of high-quality protein before a bout of resistance training exercise would lead to increases in the intra-cellular signaling involved with protein synthesis and fractional synthetic rates ("FSR" "; or directly measured muscle protein synthesis via muscle biopsy sampling) of muscle protein following the bout.

2. Different profiles of amino acid delivery would be achieved when a given amount of a high-quality protein source is consumed as a bolus (mimicking a "fast" protein) or as a series of small "pulse" feedings (mimicking a "slow" protein) starting before the commencement of a bout of resistance training.

3. Differences in the pattern of delivery of amino acids would affect intra-cellular signaling underpinning protein synthesis and the overall FSR of muscle protein following resistance training.

### Methods

Twelve resistance-trained male athletes were recruited for this study which was undertaken at the Australian Institute of Sport and approved by its Human Ethics Committee. Each athlete undertook 3 trials in a counterbalanced crossover design with a 1-2 week wash-out between trials. Each trial required subjects to complete a bout of resistance training involving single leg extensions (standardized warm-up followed by ten sets of 8-10 repetitions at a workload equivalent to 80% of the specific leg 1-repetition maximum ("1-RM") with 2 min recovery between sets). Each trial was performed on a single leg, alternating between trials. The duration of the resistance bout was ∼ 45 min.

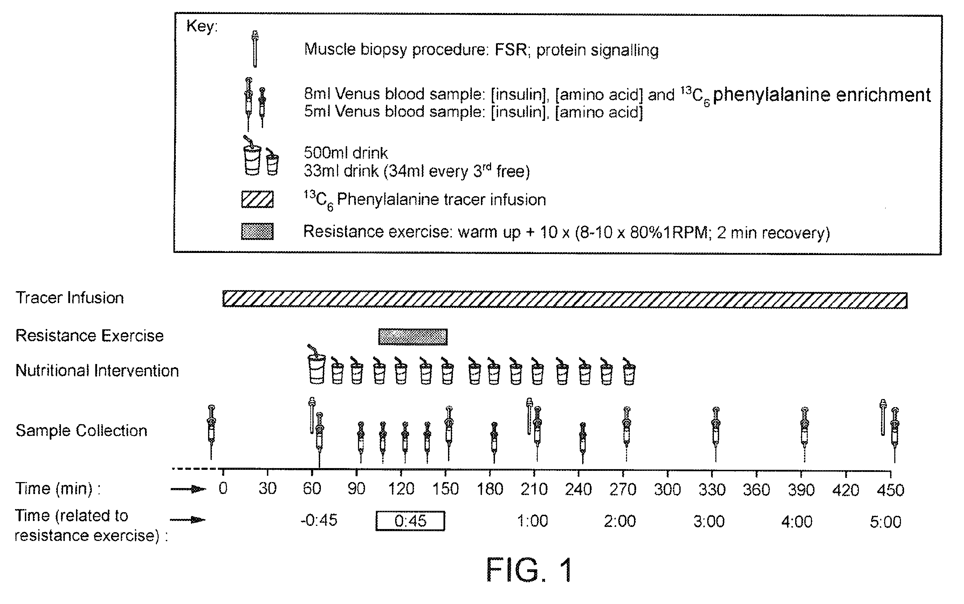

Each subject acted as their control in receiving each of three different nutritional interventions. These interventions, consisting of a series of drinks, started 45 minutes prior to the commencement of the resistance exercise session (see FIG. 1). All trials were matched for the volume of fluid (500 ml at first time point, followed by 14 x 33 ml drinks at 15 min intervals thereafter) and were identically colored and flavored with non-caloric sweetener. The three treatments varied according to the absence or presence of high quality protein dissolved in the drinks. Beverage products were provided for all experimental conditions by Nestec Ltd, Switzerland.
- BOLUS = ingestion of a bolus of 25 g of whey protein + 5 g leucine, dissolved in the first (500 ml) feeding and consumed 45 min before exercise to provide rapid and maximal amino acid delivery to the muscle. Thereafter, all drinks consumed at 15 min intervals consisted of a artificially sweetened placebo.
- PULSE = ingestion of the 25 g of whey protein + 5 g leucine divided into equal doses across each of the feedings. This protocol aimed to maintain a steady level of blood amino acids.
- PLACEBO = All treatments were artificially sweetened water: absence of feeding in association with exercise or throughout trial.

Methodologies for observing the response to resistance training and feeding involved the following protocols:
- Blood samples were collected throughout the trial to monitor patterns of plasma amino acid concentrations arising from the different protein feeding protocols.
- Muscle samples were collected by percutaneous biopsies from the vastus lateralis from a subset of subjects (n = 8) at rest and 1 hour and 5 hours after the resistance exercise. These samples were assayed for an array of signaling and transcriptional proteins involved in protein synthesis and degradation, using Real-time Polymerase-chain-reaction ("RT-PCR") and Western blotting techniques.
- Fractional Synthetic Rates (direct measurement of muscle protein synthesis) of mixed muscle protein for 5 hours following a bout of resistance training were determined from another subset of subjects from measurements of the incorporation of ¹³C₆ phenylalanine into bound muscle protein and intracellular ¹³C₆ phenylalanine enrichment as a precursor. This method involved the implementation of a primed continuous infusion of ¹³C₆ phenylalanine throughout the trials, and further measurements on the muscle samples taken from the vastus lateralis at rest and at 1 and 5 hours post-exercise.

FIG. 1 summarizes the time course of the interventions and sample collections used in our protocol

### Results

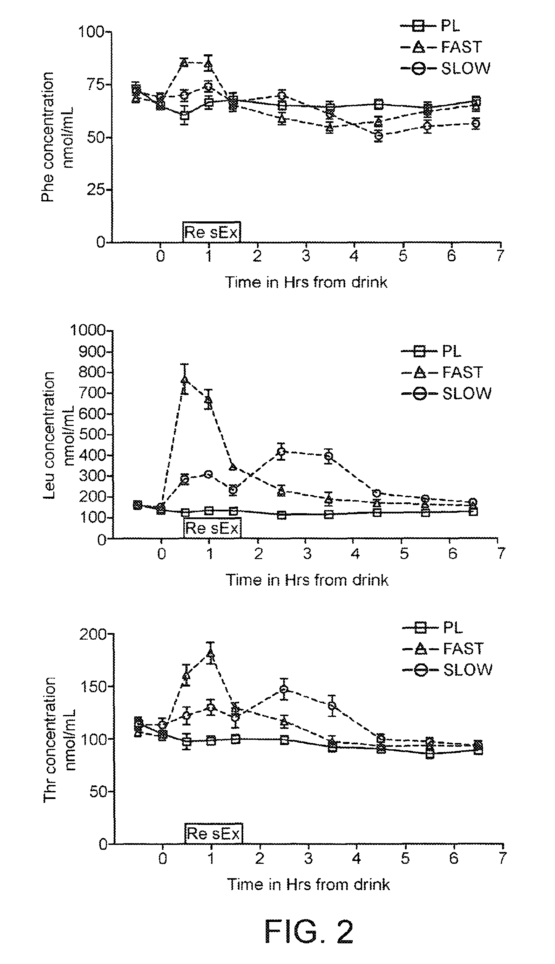

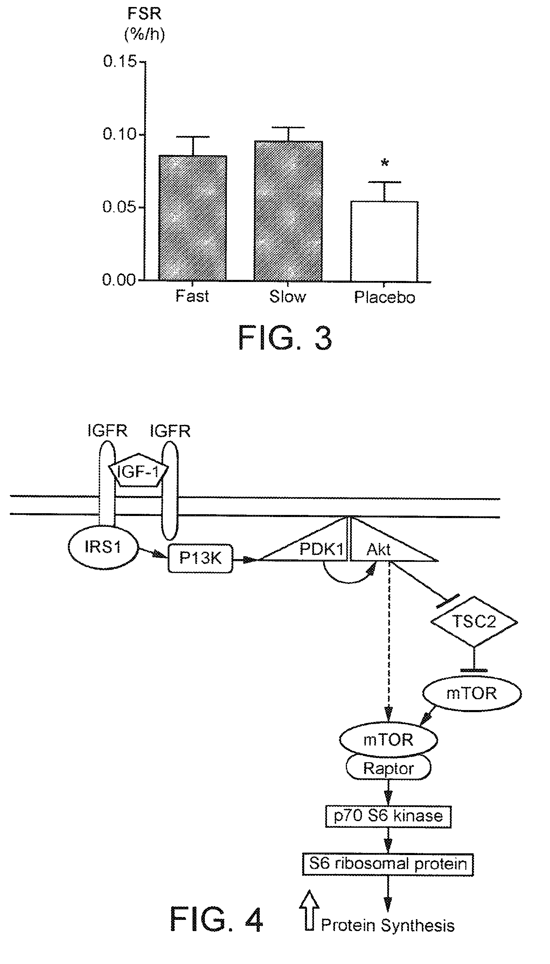

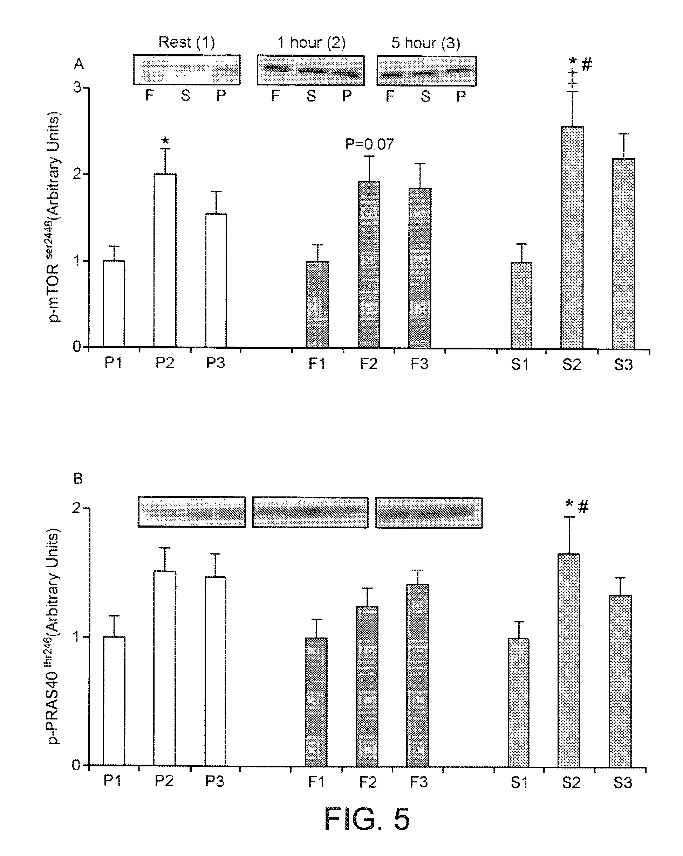

The amino acid profiles achieved by the nutritional interventions in each trial are presented in FIG. 2. The Fractional Synthetic Rates for mixed muscle protein from a subgroup of subjects are presented in FIG. 3. The array of signaling proteins measured in this study and their involvement in the anabolic pathways are summarized in FIG. 4, with individual results in FIGS. 5-6. FIG. 4 shows a schematic representation of the targeted proteins in the insulin-like growth factor (anabolic) signaling pathway. Proteins of interest highlighted by shading (PRAS40 and eEF2 kinase not shown).

FIG. 5 shows mTOR (A) and PRAS40 (B) phosphorylation at rest (1) prior to resistance training, and following 1 hour (2) and 5 hours post-exercise recovery for placebo ([P] bars), fast delivery ([F] bars) and slow delivery ([S] bars). Values are mean ± SE (mTOR n=8; PRAS40 n=7) presented in arbitrary units relative to α-tubulin. * sig diff vs. 1; 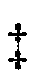 sig diff vs. 3; # sig diff vs. F2; (P <0.05).

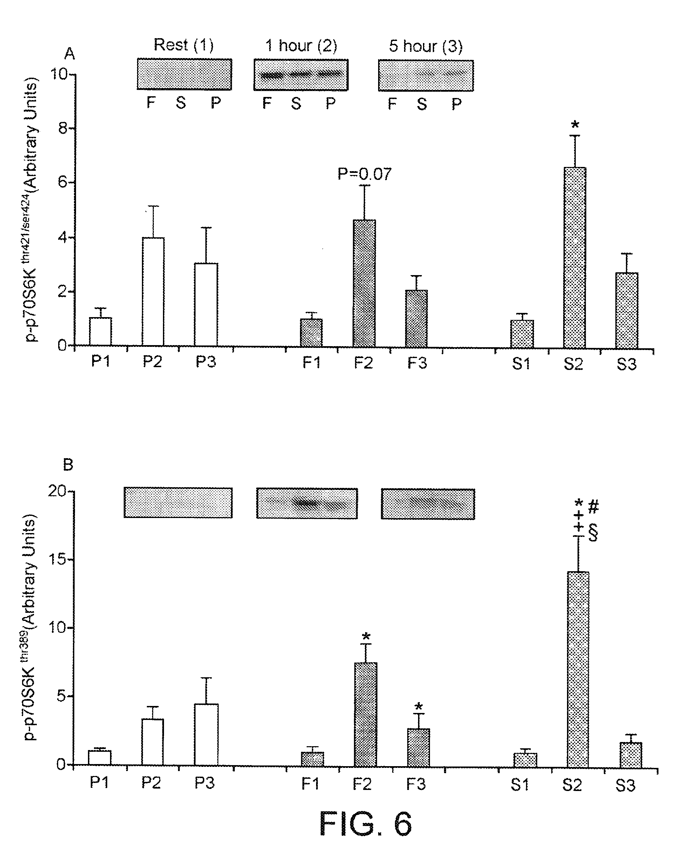

FIG. 6 shows p70 S6 kinase thr421/ser424 (A) and p70 S6 kinase ^{thr389} (B) phosphorylation at rest (1) prior to resistance training, and following 1 hour (2) and 5 hours post-exercise recovery for placebo ([P] bars), fast delivery ([F] bars) and slow delivery ([S] bars). Values are mean ± SE (n=8) presented in arbitrary units relative to α-tubulin. * sig diff vs. 1; 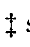 sig diffvs. 3; # sig diffvs. F2; § sig diffvs. P2 (P <0.05).

### Discussion

The following outcomes were apparent from this study:
- Manual alteration of the *timing of consumption* of a high-quality protein is effective in mimicking the plasma amino acid profiles associated with a "fast" digested protein (rapid and maximal amino acid delivery) and "slow" (sustained amino acid release) protein.
- Intake of a moderate amount (30 g) of high-quality protein (whey + added leucine) before a resistance training session is effective in increasing the Fractional Synthetic Rate of mixed muscle protein compared with a fasted condition.
- The intake of protein prior to training may differentially alter the expected pattern of delivery of amino acids after exercise. This is demonstrated most clearly in the case of plasma leucine concentrations (FIG. 2b). Although the Bolus trial in the protocol achieved a large and rapid increase in plasma amino acid concentrations following the time of ingestion, in the period immediately after the resistance exercise bout, plasma concentrations were declining and were lower than at the corresponding time-points in the Pulse trial. In contrast, the pulse pattern of intake in the Pulse trial allowed a lower but sustained increase in plasma leucine concentrations before and before and during the resistance bout, with a further increase in leucine concentrations following the completion of the exercise bout that was sustained above Bolus trial levels for 2.5 hours of recovery. It is speculated that the rise in leucine concentrations following the resistance bout may be attributed to the restoration of rates of gastric emptying and intestinal absorption that were reduced before and during the period of exercise.

A unique finding of this study was that the consumption of the protein sources ***before*** exercise was associated with a "flipping" of the characteristics of amino acid delivery in the period ***following*** exercise; the FAST protein behaved as a SLOW protein in the recovery period after exercise while the SLOW protein assumed the characteristics of a FAST protein.

The activity of key signaling and transcription factors underpinning muscle protein synthesis was increased by the resistance training with phosphorylation typically being increased at 1 hour of recovery compared with values at rest, and declining towards baseline levels by 5 hours of recovery. The feeding of protein in relation to resistance training promoted a greater increase in phosphorylation of some key proteins (Akt, mTOR, p70 S6 kinase and S6 ribosomal protein) at one hour of recovery compared with the placebo trial, with these benefits being more pronounced in the Pulse trial than the Bolus trial. These results predict a greater rate of protein synthesis with protein feedings over the fasted trial, with greater benefits in the Pulse trial.

There was no detectable difference in Fractional Synthetic Rates of mixed muscle protein between the Bolus and Pulse trials.

There are several potential reasons for incongruence in this study between activation of anabolic signaling pathways and muscle protein synthesis. These include under-powering of the calculations of muscle FSR which prevented detecting a difference between the Bolus and Pulse trials, and the difficulty in associating a rate (several hours of activity) with a cross-sectional measure of activity at a single time point. However, it is also possible that these events are not directly correlated temporarily or in magnitude

### Conclusions

Through the novel dosing patterns, effective protein synthesis was observed as fast vs. slow. This is different than the previously discussed study that found that whole-body protein synthesis was best with casein.

It should be understood that various changes and modifications to the presently preferred embodiments described herein will be apparent to those skilled in the art. Such changes and modifications can be made without departing from the scope of the present subject matter and without diminishing its intended advantages.

## Claims

1. A method of providing nutrition to an athlete, the method comprising:
providing a protein-based product; and
providing personalized guidelines for consuming the protein-based product, which provide a recommended amount of the protein-based product to consume for an exercise regimen based on one or more characteristics of the athlete and/or one or more training regimens of the athlete,
wherein the athlete consumes the protein-based product according to the personalized guidelines.

2. The method of Claim 1, wherein the protein-based products consist essentially of compositions having the same protein profile but differing in dose.

3. The method of Claim 1, wherein the protein-based products comprise products having the same amino acid profile but differing in dose.

4. The method of any one of the preceding claims, wherein the personalized guidelines recommend a bolus feeding dose before beginning the exercise regimen.

5. The method of any one of Claims 1 to 3, wherein the personalized guidelines recommend a pulse feeding dose before beginning, throughout, and after the exercise regimen.

6. The method of any one of the preceding claims, wherein the characteristic of the athlete is selected from the group consisting of weight, height, age, gender and combinations thereof.

7. The method of any one of the preceding claims, wherein the training regimen is selected from the group consisting of aerobics, weight-lifting, running, swimming, cycling, team sports and combinations thereof

8. The method of any one of the preceding claims, wherein the personalized guidelines are further based on the duration of the training program of the athlete.

9. The method of any one of Claims 1 to 7, wherein the personalized guidelines are determined based on a sport.

10. The method of any one of the preceding claims, wherein the training program is designed to achieve an objective selected from the group consisting of weight-loss, muscle building and combinations thereof.

11. The method of any one of the preceding claims, wherein the personalized guidelines provide the recommended amount of the protein-based product to consume before and during exercising based on at least one of the characteristic of the athlete and the training program of the athlete.

12. The method of any one of the preceding claims, wherein the dose of product weighs from about 5 grams to about 35 grams.

13. The method of any one of the preceding claims, wherein the protein-based product comprises a protein blend comprising soy protein isolates, whey protein isolates and calcium caseinate.

14. A method of increasing the benefit of resistance exercise, the method comprising:
providing at least two different doses of a protein composition that has substantially the same amino acid profile; and
providing guidelines as to which dose to take pre-exercise, during exercise, and post exercise,
wherein the doses of protein composition are consumed according to the guidelines.

## Patentansprüche

1. Verfahren zur Bereitstellung von Ernährung für einen Sportler, mit folgenden Schritten:
Bereitstellen eines Produkts auf Eiweißbasis; sowie
Bereitstellen von individuell angepassten Richtlinien für den Verzehr des Produkts auf Eiweißbasis, welche eine empfohlene Menge des Produkts auf Eiweißbasis zum Verzehr für einen Übungsplan vorsehen, der auf einer oder mehreren Eigenschaften des Sportlers und/oder einem oder mehreren Trainingsplänen des Sportlers basiert,
wobei der Sportler das Produkt auf Eiweißbasis gemäß den individuell angepassten Richtlinien verzehrt.

2. Verfahren gemäß Anspruch 1, wobei die Produkte auf Eiweißbasis im Wesentlichen aus Zusammensetzungen bestehen, die dasselbe Eiweißprofil, aber in unterschiedlichen Mengen aufweisen.

3. Verfahren gemäß Anspruch 1, wobei die Produkte auf Eiweißbasis Produkte umfassen, die dasselbe Aminosäureprofil, aber in unterschiedlichen Mengen aufweisen.

4. Verfahren gemäß einem der vorhergehenden Ansprüche, wobei die individuell angepassten Richtlinien eine Bolus-Nahrungsmenge vor dem Beginn des Übungsplans empfehlen.

5. Verfahren gemäß einem der Ansprüche 1 bis 3, wobei die individuell angepassten Richtlinien eine wiederholte Nahrungsmenge vor, während und nach dem Übungsplan empfehlen.

6. Verfahren gemäß einem der vorhergehenden Ansprüche, wobei die Eigenschaft des Sportlers aus der Gruppe bestehend aus Gewicht, Größe, Alter, Geschlecht und Kombinationen aus diesen ausgewählt wird.

7. Verfahren gemäß einem der vorhergehenden Ansprüche, wobei der Übungsplan aus der Gruppe bestehend aus Aerobic, Gewichtheben, Laufen, Schwimmen, Zirkeltraining, Teamsportarten und Kombinationen aus diesen ausgewählt wird.

8. Verfahren gemäß einem der vorhergehenden Ansprüche, wobei die individuell angepassten Richtlinien weiterhin auf der Dauer des Trainingsprogramms des Sportlers basieren.

9. Verfahren gemäß einem der Ansprüche 1 bis 7, wobei die individuell angepassten Richtlinien auf der Grundlage einer Sportart festgelegt werden.

10. Verfahren gemäß einem der vorhergehenden Ansprüche, wobei das Trainingsprogramm zum Erreichen eines Zieles ausgewählt aus der Gruppe bestehend aus Gewichtsabnahme, Muskelaufbau und Kombinationen aus diesen ausgelegt ist.

11. Verfahren gemäß einem der vorhergehenden Ansprüche, wobei die individuell angepassten Richtlinien die empfohlene Menge des Produkts auf Eiweißbasis vorsehen, die vor und während der Sportausübung auf der Grundlage von mindestens entweder der Eigenschaft des Sportlers oder dem Trainingsprogramm des Sportlers zu verzehren ist.

12. Verfahren gemäß einem der vorhergehenden Ansprüche, wobei die Produktmenge zwischen ca. 5 Gramm und ca. 35 Gramm beträgt.

13. Verfahren gemäß einem der vorhergehenden Ansprüche, wobei das Produkt auf Eiweißbasis eine Eiweißmischung aufweist, die Sojaeiweißisolate, Molkeeiweißisolate und Kalziumkaseinat umfasst.

14. Verfahren zur Steigerung der Vorteile von Krafttraining, mit den folgenden Schritten:
Vorsehen von mindestens zwei unterschiedlichen Mengen einer Eiweißzusammensetzung, die im Wesentlichen dasselbe Aminosäureprofil aufweist; sowie
Vorsehen von Richtlinien bezüglich der Menge, die vor dem Training, während des Trainings und nach dem Training einzunehmen ist,
wobei die Mengen der Eiweißzusammensetzung gemäß den Richtlinien verzehrt werden.

## Revendications

1. Procédé pour la nutrition d'un athlète, le procédé comprenant :
la fourniture d'un produit à base de protéines ; et
la fourniture de directives personnalisées pour la consommation du produit à base de protéines, qui prévoient une quantité recommandée du produit à base de protéines à consommer pour un programme d'exercice basé sur une ou plusieurs caractéristiques de l'athlète et/ou un ou plusieurs programmes d'entraînement de l'athlète,
dans lequel l'athlète consomme le produit à base de protéines selon les directives personnalisées.

2. Procédé selon la revendication 1, dans lequel les produits à base de protéines sont constitués essentiellement de compositions présentant le même profil protéinique mais une dose différente.

3. Procédé selon la revendication 1, dans lequel les produits à base de protéines comprennent des produits présentant le même profil d'acides aminés mais une dose différente.

4. Procédé selon l'une quelconque des revendications précédentes, dans lequel les directives personnalisées recommandent une dose d'alimentation bolus avant de commencer le programme d'exercice.

5. Procédé selon l'une quelconque des revendications 1 à 3, dans lequel les directives personnalisées recommandent une dose d'alimentation intermittente avant de commencer, pendant, et après le programme d'exercice.

6. Procédé selon l'une quelconque des revendications précédentes, dans lequel la caractéristique de l'athlète est sélectionnée parmi le groupe constitué par le poids, la taille, l'âge, le sexe et des combinaisons de ceux-ci.

7. Procédé selon l'une quelconque des revendications précédentes, dans lequel le programme d'entraînement est sélectionné parmi le groupe constitué par l'aérobic, la musculation, la course à pied, la natation, le cyclisme, les sports d'équipe et des combinaisons de ceux-ci.

8. Procédé selon l'une quelconque des revendications précédentes, dans lequel les directives personnalisées sont en outre basées sur la durée du programme d'entraînement de l'athlète.

9. Procédé selon l'une quelconque des revendications 1 à 7, dans lequel les directives personnalisées sont déterminées sur la base d'un sport.

10. Procédé selon l'une quelconque des revendications précédentes, dans lequel le programme d'entraînement est conçu pour atteindre un objectif sélectionné parmi le groupe constitué par la perte de poids, le renforcement musculaire et des combinaisons de ceux-ci.

11. Procédé selon l'une quelconque des revendications précédentes, dans lequel les directives personnalisées fournissent la quantité recommandée du produit à base de protéines à consommer avant et pendant l'exercice sur la base d'au moins soit la caractéristique de l'athlète soit le programme d'entraînement de l'athlète.

12. Procédé selon l'une quelconque des revendications précédentes, dans lequel la dose de produit pèse entre environ 5 grammes et environ 35 grammes.

13. Procédé selon l'une quelconque des revendications précédentes, dans lequel le produit à base de protéines comprend un mélange de protéines comprenant des isolats de protéine, de soja, des isolats de protéine de lactosérum et du caséinate de calcium.

14. Procédé pour augmenter le bénéfice d'exercices de résistance, le procédé comprenant :
la fourniture d'au moins deux doses différentes d'une composition de protéines qui présente sensiblement le même profil d'acides aminés ; et
la fourniture de directives relatives à la dose à prendre avant l'exercice, pendant l'exercice et après l'exercice,
dans lequel les doses de la composition de protéines sont consommées selon les directives.
